# NEUE EUROPÄISCHE PATENTSCHRIFT

(11) **EP 1 841 797 B2**
(45) Veröffentlichungstag und Bekanntmachung des Hinweises auf die Entscheidung über den Einspruch: **15.04.2015**
(45) Hinweis auf die Patenterteilung: 27.04.2011
(21) Anmeldenummer: 06700825.0
(22) Anmeldetag: 14.01.2006
(51) Int. Cl.: C08F 2/00, C08F 20/06, A61L 15/60, A61L 15/24

(54) **VERFAHREN ZUR HERSTELLUNG VON POLYMEREN DURCH SPRÜHPOLYMERISATION**
METHOD FOR PRODUCING POLYMERS BY MEANS OF SPRAY POLYMERISATION
PROCEDE DE PRODUCTION DE POLYMERES PAR POLYMERISATION PAR PULVERISATION

(30) Priorität: 18.01.2005 DE 102005002412
(43) Veröffentlichungstag der Anmeldung: 10.10.2007
(73) Patentinhaber: BASF SE, 67056 Ludwigshafen (DE)
(72) Erfinder: LÖSCH, Dennis, 67122 Altrip (DE); SEIDL, Volker, 68163 Mannheim (DE); STUEVEN, Uwe, 65812 Bad Soden (DE)
(86) Internationale Anmeldenummer: PCT/EP2006/000289
(87) Internationale Veröffentlichungsnummer: WO 2006/077054

(56) Entgegenhaltungen:
- EP-A- 0 816 383
- WO-A-2005/030810
- US-A- 5 506 324
- US-A- 5 532 323
- US-A- 6 143 821
- PATENT ABSTRACTS OF JAPAN Bd. 013, Nr. 372 (C-627), 17. August 1989 (1989-08-17) & JP 01 126314 A (NIPPON SHOKUBAI KAGAKU KOGYO CO LTD), 18. Mai 1989 (1989-05-18)

## Beschreibung

Die vorliegende Erfindung betrifft ein Verfahren zur Herstellung wasserabsorbierender Polymerpartikel durch Sprühpolymerisation einer Monomerlösung, die wasserabsorbierenden Polymerpartikel selber sowie Hygieneartikel, enthaltend diese wasserabsorbierenden Polymerpartikel.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils angegebenen Kombination, sondern auch in anderen Kombinationen verwendbar sind, ohne den Rahmen der Erfindung zu verlassen.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Eigenschaften der wasserabsorbierenden Polymer können über den Vernetzungsgrad eingestellt werden. Mit steigendem Vernetzungsgrad steigt die Gelfestigkeit und sinkt die Absorptionskapazität. Dies bedeutet, dass mit steigender Absorption unter Druck (AUL) die Zentrifugenretentionskapazität (CRC) abnimmt (zu sehr hohen Vernetzungsgraden nimmt auch die Absorption unter Druck wieder ab).

Zur Verbesserung der Anwendungseigenschaften, wie beispielsweise Flüssigkeitsleitfähigkeit (SFC) in der Windel und Absorption unter Druck (AUL), werden wasserabsorbierende Polymerpartikel im allgemeinen nachvernetzt. Dadurch steigt nur der Vernetzungsgrad der Partikeloberfläche, wodurch die Absorption unter Druck (AUL) und die Zentrifugenretentionskapazität (CRC) zumindest teilweise entkoppelt werden können. Diese Nachvernetzung kann in wässriger Gelphase durchgeführt werden. Vorzugsweise werden aber gemahlene und abgesiebte Polymerpartikel (Grundpolymer) an der Oberfläche mit einem Nachvernetzer beschichtet, getrocknet und thermisch nachvernetzt. Dazu geeignete Vernetzer sind Verbindungen, die mindestens zwei Gruppen enthalten, die mit den Carboxylatgruppen des hydrophilen Polymeren kovalente Bindungen bilden können.

Die Nachvernetzung wird in der Monographie "Modern Superabsorbent Polymer Technology", F.L. Buchholz und A.T. Graham, Wiley-VCH, 1998, Seiten 97 bis 103, beschrieben. Üblicherweise werden die wasserabsorbierenden Polymerpartikel mit dem Nachvernetzer benetzt und thermisch nachvernetzt, wobei die Polymerpartikel mittels heißer Luft oder mittels Kontakttrocknung erwärmt und gleichzeitig getrocknet werden.

Nachteilig bei den bisher üblichen Verfahren ist, dass die Polymerisation, die Trocknung und die Klassierung des Grundpolymers sowie die thermische Nachvernetzung in getrennten Verfahrenschritten durchgeführt werden müssen.

Durch Sprühpolymerisation konnten die Verfahrensschritte Polymerisation und Trocknung zusammengefasst werden. Zusätzlich konnte die Partikelgröße durch geeignete Verfahrensführung in gewissen Grenzen eingestellt werden.

Die Herstellung wasserabsorbierender Polymerpartikel durch Sprühpolymerisation wird beispielsweise in EP-A-0 348 180, WO-A-96/40427 sowie den älteren deutschen Patentanmeldungen mit den Aktenzeichen DE-10340253 und DE-102004024437 beschrieben.

Nachteilig bei den bisherigen Sprühpolymerisationsverfahren ist aber, dass weiterhin ein zusätzlicher Nachvemetzungsschritt erforderlich ist. Ohne diesen zusätzliche Schritt konnten keine wasserabsorbierenden Polymerpartikel mit ausreichender Flüssigkeitsweiterleitung (SFC) und Absorption unter Druck (AUL) erhalten werden.

Aufgabe der vorliegenden Erfindung war daher die Bereitstellung eines verbesserten Verfahrens zur Herstellung wasserabsorbierender Polymerpartikel.

Ausgehend von einer Monomerlösung sollten in einem Verfahrensschritt wasserabsorbierende Polymerpartikel erhalten werden, die im Bereich der Partikeloberfläche höher vernetzt sind als im Partikelinnern.

Weiterhin sollten ausgehend von einer Monomerlösung in einem Verfahrensschritt wasserabsorbierende Polymerpartikel mit verbesserter Absorption unter Druck (AUL) und hoher Zentrifugenretentionskapazität (CRC) erhalten werden.

Die erfindungsgemäßen wasserabsorbierende Polymerpartikel können durch Polymerisation einer Monomerlösung, enthaltend
a) mindestens ein ethylenisch ungesättigtes, säuregruppentragendes Monomer,
b) mindestens einen Vernetzer,
b') mindestens einen weiteren Vernetzer,
c) gegebenenfalls ein oder mehrere mit dem Monmeren a) copolymerisierbare ethylenisch und/oder allylisch ungesättigte Monomere und
d) gegebenenfalls ein oder mehrere wasserlösliche Polymere, auf die die Monomere a), b) und ggf. c) zumindest teilweise aufgepfropft werden können,
wobei die Monomerlösung versprüht wird, dadurch gekennzeichnet, dass sich der mindestens eine Vernetzer b) an der Tropfenoberfläche anreichert und der mindestens eine weitere Vermetzer b') mindestens zwei polymrisierbare Gruppen aufweist, die in das Polymemetzwerk radikalisch einpolymerisiert werden können, hergestellt werden.

Die Anreicherungsgeschwindigkeit des Vernetzers b) wird durch Messungen der dynamischen Oberflächenspannung in der Monomerlösung gemäß ASTM D3825 bestimmt. Dazu werden Gasblasen in der Monomerlösung erzeugt und die Oberflächenspannung der Gasblasen in Abhängigkeit der Blasenlebensdauer ermittelt. Durch Diffusion des Vernetzers b) verändert sich die Oberflächenspannung (dynamische Oberflächenspannung) bis der Gleichgewichtszustand erreicht ist (statische Oberflächenspannung). Die Zeit bis zur Gleichgewichtseinstellung ist ein Maß dafür, wie schnell sich der Vernetzer b) an der Phasengrenzfläche anreichert und beträgt üblicherweise weniger als 10 Sekunden, vorzugsweise weniger als 5 Sekunden, besonders bevorzugt weniger als eine Sekunde. Das Gleichgewicht gilt als erreicht, wenn die weitere Änderung weniger als 5% der Gesamtänderung beträgt.

Die Vernetzer b) enthalten vorzugsweise mindestens zwei radikalisch polymerisierbare Gruppen.

Geeignete Vernetzer b) sind Vernetzer die hydrophobe und hydrophile Gruppen aufweisen, beispielsweise hydrophob modifizierte hydrophile Vernetzer. Vorzugsweise weisen die hydrophilen Reste die polymerisierbaren Gruppen auf.

Ausgangsstoffe für die Vernetzer b) können Polyole sein, die sich bereits selber beim Versprühen in wäßriger Lösung an der Tropfenoberfläche anreichern. Beispiele hierfür sind Blockpolymere auf Basis von Propylenoxid und Ethylenoxid.

Bevorzugte Vernetzer b) sind Vernetzer der allgemeinen Formel I 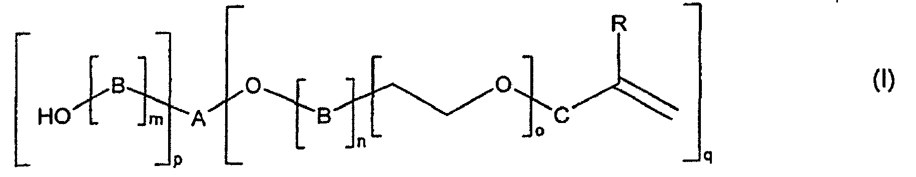 in der
- R: für gleiche und verschiedene Reste, ausgewählt aus der Gruppe Wasser- stoff, Methyl und Ethyl steht,
- A: ein C₃- bis C₂₀-Alk(p+q)yl oder C₃- bis C₂₀-Heteroalk(p+q)yl bedeutetet,
- B: für gleiche oder verschiedene Gruppen, ausgewählt aus 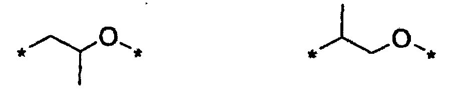 steht und mit * die Verknüpfungspositionen gekennzeichnet sind,
- C: für gleiche oder verschiedene Gruppen, ausgewählt aus Carbonyl und Methylen, steht,
- m, n und o: eine ganze Zahl von 0 bis 100 bedeuten,
- p: eine ganze Zahl von 0 bis 6 bedeutet und
- q: eine ganze Zahl von 2 bis 6 bedeutet,
wobei p+q vorzugsweise eine ganze Zahl von 2 bis 6 ist.

Besonders bevorzugte Vernetzer b) sind Vernetzer der allgemeinen Formel I in der
- R: für gleiche und verschiedene Reste, ausgewählt aus der Gruppe Wasser- stoff und Methyl steht,
- A: ein C₃- bis C₁₀-Alk(p+q)yl oder C₃- bis C₁₀-Heteroalk(p+q)yl bedeutetet,
- B: für gleiche oder verschiedene Gruppen, ausgewählt aus 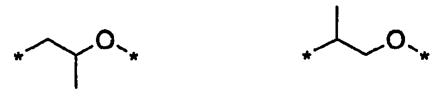 steht und mit * die Verknüpfungspositionen gekennzeichnet sind,
- C: für gleiche oder verschiedene Gruppen, ausgewählt aus Carbonyl und Methylen, steht,
- m, n und o: eine ganze Zahl von 1 bis 50 bedeuten,
- p: eine ganze Zahl von 0 bis 4 bedeutet und
- q: eine ganze Zahl von 2 bis 4 bedeutet,
wobei p+q vorzugsweise eine ganze Zahl von 2 bis 4 ist.

Ganz besonders bevorzugte Vernetzer b) sind Vernetzer der allgemeinen Formel I in der
- R: für Wasserstoff steht,
- A: ein C₃- bis C₆-Alk(p+q)yl oder C₃- bis C₆-Heteroalk(p+q)yl bedeutetet,
- B: für gleiche oder verschiedene Gruppen, ausgewählt aus 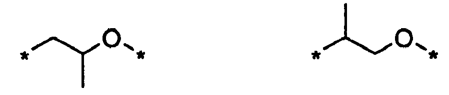 steht und mit * die Verknüpfungspositionen gekennzeichnet sind,
- C: für gleiche oder verschiedene Gruppen, ausgewählt aus Carbonyl und Methylen, steht,
- m, n und o: eine ganze Zahl von 2 bis 30 bedeuten,
- p: eine ganze Zahl von 0 bis 2 bedeutet und
- q: 2 oder 3 bedeutet,
wobei p+q vorzugsweise 2 oder 3 ist.

Weiterhin bevorzugte Vernetzer b) sind Vernetzer der allgemeinen Formel II 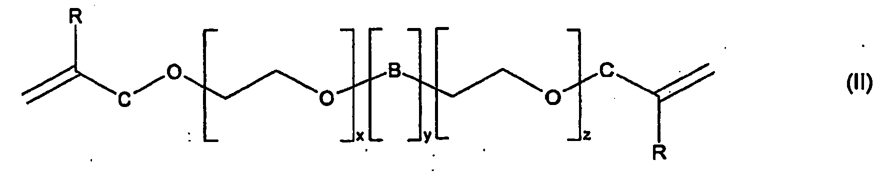 in der R, B und C die obengenannten Bedeutungen haben und vorzugsweise
- x und z: gleich oder verschieden sind und eine ganze Zahl von 0 bis 100 bedeuten und
- y: eine ganze Zahl von 1 bis 200 bedeutet,
besonders bevorzugt
- x und z: gleich oder verschieden sind und eine ganze Zahl von 1 bis 50 bedeuten und
- y: eine ganze Zahl von 3 bis 100 bedeutet,
und ganz besonders bevorzugt
- x und z: gleich oder verschieden sind und eine ganze Zahl von 2 bis 30 bedeuten und
- y: eine ganze Zahl von 5 bis 60 bedeutet.

Geeignete Monomere a) sind beispielsweise ethylenisch ungesättigte Carbonsäuren, wie Acrylsäure, Methacrylsäure, Maleinsäure, Fumarsäure und Itaconsäure, oder deren Derivate, wie Acrylamid, Methacrylamid, Acrylsäureester und Methacrylsäureester. Besonders bevorzugte Monomere sind Acrylsäure und Methacrylsäure. Ganz besonders bevorzugt ist Acrylsäure.

Die Säuregruppen der Monomere a) sind üblicherweise teilweise neutralisiert, vorzugsweise zu 25 bis 85 mol-%, bevorzugt zu 27 bis 80 mol-%, besonders bevorzugt zu 27 bis 30 mol-% oder 40 bis 75 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung, als Schmelze, oder bevorzugt auch als Feststoff in die Monomerlösung erreicht. Beispielsweise kann Natriumhydroxid mit einem Wasseranteil deutlich unter 50 Gew.% als wachsartige Masse mit einem Schmelzpunkt oberhalb 23°C vorliegen. In diesem Fall ist eine Dosierung als Stückgut oder Schmelze bei erhöhter Temperatur möglich.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden 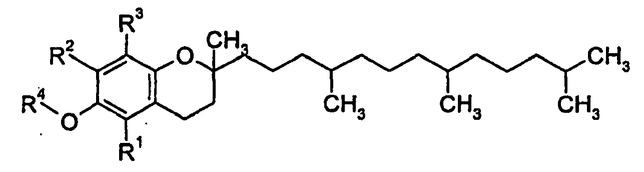 wobei R¹ Wasserstoff oder Methyl, R² Wasserstoff oder Methyl, R³ Wasserstoff oder Methyl und R⁴ Wasserstoff oder ein Säurerest mit 1 bis 20 Kohlenstoffatomen bedeutet.

Bevorzugte Reste für R⁴ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit R¹ = R² = R³ = Methyl, insbesondere racemisches alpha-Tocopherol. R¹ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindesten 30 Gew.-ppm, besonders bevorzugt mindesten 30 Gew.-ppm, inbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenen Gehalt an Hydrochinonhalbether verwendet werden.

Die Monomerlösung enthält neben den Vernetzern b) noch weitere Vernetzer b'). Die Vernetzer b') sind Verbindungen mit mindestens zwei polymerisierbaren Gruppen, die in das Polymernetzwerk radikalisch einpolymerisiert werden können. Durch Verwendung der Vernetzer b') in Kombination mit den Vernetzern b) kann der Vernetzungsgrad im Bereich der Partikeloberfläche und im Partikelinnern unabhängig voneinander optimiert werden. Geeignete Vernetzer b') sind beispielsweise Ethylenglykoldimethacrylat, Diethylenglykoldiacrylat, Allylmethacrylat, Trimethylolpropantriacrylat, Triallylamin, Tetraallyloxyethan, wie in EP-A-0 530 438 beschrieben, Di- und Triacrylate, wie in EP-A-0 547 847, EP-A-0 559 476, EP-A-0 632 068, WO-A-93/21237, WO-A-03/104299, WO-A-03/104300, WO-A-03/104301 und in der deutschen Patentanmeldung mit dem Aktenzeichen 10331450.4 beschrieben, gemischte Acrylate, die neben Acrylatgruppen weitere ethylenisch ungesättigte Gruppen enthalten wie in den deutschen Patentanmeldungen mit den Aktenzeichen DE-10331456 und DE-10355401 beschrieben, oder Vernetzermischungen, wie beispielsweise in DE-A-195 43 368, DE-A-196 46 484, WO-A-90/15830 und WO-A-02/32962 beschrieben.

Geeignete Vernetzer b') sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Weiterhin geeignete Vernetzer b') sind Pentaerythritoldi- Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer b') sind jedoch Di- und Triacrylate des 3- bis 15-fach ethoxylierten Glyzerins, des 3- bis 15-fach ethoxylierten Trimethylolpropans, des 3- bis 15-fach ethoxylierten Trimethylolethans, inbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des 40-fach ethoxylierten Glyzerins, Trimethylolethans oder Trimethylolpropans.

Ganz besonders bevorzugte Vernetzer b') sind die mit Acrylsäure oder Methacrylsäure zu Di- oder Triacrylaten veresterten mehrfach ethoxylierten und/oder propoxylierten Glyzerine wie sie beispielsweise in der älteren deutschen Anmeldung mit Aktenzeichen DE 10319462.2 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5- fach ethoxylierten und/oder propoxylierten Glyzerins. Am meisten bevorzugt sind die Triacrylate des 3- bis 5-fach ethoxylierten und/oder propoxylierten Glyzerins. Diese zeichnen sich durch besonders niedrige Restgehalte (typischerweise unter 10 Gew.-ppm) im wasseraborbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Mit den Monomeren a) copolymerisierbare ethylenisch ungesättigte Monomere c) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Als wasserlösliche Polymere d) können Polyvinylalkohol, Polyvinylpyrrolidon, Stärke, Stärkederivate, Polyglykole oder Polyacrylsäuren, vorzugsweise Polyvinylalkohol und Stärke, eingesetzt werden.

Die Reaktion kann in Gegenwart eines inerten Trägergases durchgeführt werden, wobei inert bedeutet, dass das Trägergas mit den Bestandteilen der Monomerlösung nicht reagieren kann. Das inerte Trägergas ist vorzugsweise Stickstoff. Der Sauerstoffgehalt des inerten Trägergases beträgt vorteilhaft unter 1 Vol.-%, vorzugsweise unter 0,5 Vol.-%, besonders bevorzugt unter 0,1 Vol.-%.

Das inerte Trägergas kann im Gleichstrom oder im Gegenstrom zu den frei fallenden Tropfen der Monomerlösung durch den Reaktionsraum geführt werden, bevorzugt im Gleichstrom. Vorzugsweise wird das Trägergas nach einem Durchgang zumindest teilweise, bevorzugt zu mindestens 50%, besonders bevorzugt zu mindestens 75%, als Kreisgas in den Reaktionsraum zurückgeführt. Üblicherweise wird eine Teilmenge des Trägergases nach jedem Durchgang ausgeschleust, vorzugsweise mindestens 10%.

Die Gasgeschwindigkeit wird vorzugsweise so eingestellt, dass die Strömung im Reaktor gerichtet ist, beispielsweise liegen keine der allgemeinen Strömungsrichtung entgegengesetzte Konvektionswirbel vor, und beträgt beispielsweise 0,02 bis 1,5 m/s, bevorzugt 0,05 bis 0,4 m/s.

Die Reaktionstemperatur beträgt vorzugsweise 70 bis 250°C, besonders bevorzugt 80 bis 190°C, ganz besonders bevorzugt 90 bis 160°C.

Die Konzentration der Monomeren a) in der Monomerlösung beträgt üblicherweise 2 bis 80 Gew.-%, vorzugsweise 5 bis 70 Gew.-%, besonders bevorzugt 10 bis 60 Gew.-%.

Die Löslichkeit der Monomeren a) in Wasser beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 50 g/100 g Wasser.

Die bevorzugten Polymerisationsinhibitoren benötigen für eine optimale Wirkung gelösten Sauerstoff. Daher können die Polymerisationsinhibitoren vor der Polymerisation durch Inertisierung, d.h. Durchströmen mit einem inerten Gas, vorzugsweise Stickstoff, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Monomeren werden in wässriger Lösung in Gegenwart von Initiatoren miteinander polymerisiert.

Die Initiatoren werden in üblichen Mengen eingesetzt, beispielsweise in Mengen von 0,001 bis 5 Gew.-%, vorzugsweise 0,01 bis 1 Gew.-%, bezogen auf die zu polymerisierenden Monomere.

Als Initiatoren können sämtliche unter den Polymerisationsbedingungen in Radikale zerfallende Verbindungen eingesetzt werden, beispielsweise Peroxide, Hydroperoxide, Wasserstoffperoxid, Persulfate, Azoverbindungen und die sogenannten Redoxinitiatoren. Bevorzugt ist der Einsatz von wasserlöslichen Initiatoren. In manchen Fällen ist es vorteilhaft, Mischungen verschiedener Initiatoren zu verwenden, beispielsweise Mischungen aus Wasserstoffperoxid und Natrium- oder Kaliumperoxodisulfat. Mischungen aus Wasserstoffperoxid und Natriumperoxodisulfat können in jedem beliebigen Verhältnis verwendet werden.

Geeignete organische Peroxide sind beispielsweise Acetylacetonperoxid, Methylethylketonperoxid, tert.-Butylhydroperoxid, Cumolhydroperoxid, tert.-Amylperpivalat, tert.-Butylperpivalat, tert.-Butylperneohexanoat, tert.-Butylperisobutyrat, tert.-Butyl-per-2-ethylhexanoat, tert.-Butylperisononanoat, tert.-Butylpermaleat, tert.-Butylperbenzoat, Di-(2-ethylhexyl)peroxydicarbonat, Dicyclohexylperoxydicarbonat, Di-(4-tert.-butylcyclohexyl)peroxydicarbonat, Dimyristilperoxydicarbonat, Diacetylperoxydicarbonat, Allylperester, Cumylperoxyneodecanoat, tert.-Butylper-3,5,5-trimethylhexanoat, Acetylcyclohexylsulfonylperoxid, Dilaurylperoxid, Dibenzoylperoxid und tert.-Amylperneodekanoat.

Bevorzugte Initiatoren sind Azoverbindungen, beispielsweise 2,2'-Azobis-isobutyronitril, 2,2'-Azobis(2,4-dimethylvaleronitril) und 2,2'-Azobis(4-methoxy-2,4-dimethylvaleronitril), insbesondere wasserlösliche Azostarter, beispielsweise 2,2'-Azobis{2-[1-(2-hydroxyethyl)-2-imidazolin-2-yl]propan}dihydrochlorid, 2,2'-Azobis-(2-amidinopropan)dihydrochlorid, 2,2'-Azobis[2-(2-imidazolin-2-yl)propan]dihydrochlorid und 2,2'-Azobis[2-(5-methyl-2-imidazolin-2-yl)propan]dihydrochlorid. Ganz besonders bevorzugt sind 2,2'-Azobis[2-(2-imidazolin-2-yl)propan]dihydrochlorid und 2,2'-Azobis[2-(5-methyl-2-imidazolin-2-yl)propan]dihydrochlorid.

Weiterhin bevorzugte Initiatoren sind außerdem Redoxinitiatoren. Die Redoxinitiatoren enthalten als oxidierende Komponente mindestens eine der oben angegebenen Peroxoverbindungen und als reduzierende Komponente beispielsweise Ascorbinsäure, Glukose, Sorbose, Ammonium- oder Alkalimetallhydrogensulfit, -sulfit, -thiosulfat, - hyposulfit, -pyrosulfit, -sulfid oder Natriumhydroxymethylsulfoxylat. Vorzugsweise verwendet man als reduzierende Komponente des Redoxkatalysators Ascorbinsäure oder Natriumpyrosulfit. Bezogen auf die bei der Polymerisation eingesetzte Menge an Monomeren verwendet man beispielsweise 1 x 10⁻⁵ bis 1 Mol-% der reduzierenden Komponente des Redoxkatalysators.

Besonders bevorzugt wird die Polymerisation durch Einwirkung energiereicher Strahlung ausgelöst, wobei man üblicherweise sogenannte Photoinitiatoren als Initiator verwendet. Hierbei kann es sich beispielsweise um sogenannte α-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen, wie die oben genannten Radikalbildner, substituierte Hexaarylbisimidazole oder Acylphosphinoxide, insbesondere 2-Hydroxy-2-methylpropiophenon (Darocure® 1173). Beispiele für Azide sind 2-(N,N-Dimethylamino)-ethyl-4-azidocinnamat, 2-(N,N-Dimethyl-amino)-ethyl-4-azidonaphthylketon, 2-(N,N-Dimethylamino)-ethyl-4-azidobenzoat, 5-Azido-1-naphthyl-2'-(N,N-dimethylamino)ethylsulfon, N-(4-Sulfonylazidophenyl).maleinimid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclohexanon und 2,6-Bis-(p-azidobenzyliden)-4-methylcyclohexanon.

Besonders bevorzugte Initiatoren sind Azoinitiatoren, wie 2,2'-Azobis[2-(2-imidazolin-2-yl)propan]dihydrochlorid und 2,2'-Azobis[2-(5-methyl-2-imidazolin-2-yl)propan]dihydrochlorid, und Photoinitiatoren, wie 2-Hydroxy-2-methylpropiophenon und 1-[4-(2-Hydroxyethoxy)-phenyl]-2-hydroxy-2-methyl-1-propan-1-on, Redoxinitiatoren, wie Natriumpersulfat/Hydroxymethylsulfinsäure, Ammoniumperoxodisulfat/Hydroxymethylsulfinsäure, Wasserstoffperoxid/Hydroxymethylsulfinsäure, Natriumpersulfat/Ascorbinsäure, Ammoniumperoxodisulfat/Ascorbinsäure und Wasserstoffperoxid/Ascorbinsäure, Photoinitiatoren, wie 1-[4-(2-Hydroxyethoxy)-phenyl]-2-hydroxy-2-methyl-1-propan-1-on, sowie deren Mischungen.

Der pH-Wert der Monomerlösung ist nicht entscheidend. Entsprechend der Produktanforderungen kann aber der pH-Wert des erfindungsgemäßen Polymeren über den pH-Wert der Monomerlösung auf den gewünschten Bereich eingestellt werden. Beispielsweise sollten Polymere für kosmetische Anwendungen üblicherweise einen pH-Wert von 5 bis 6 aufweisen.

Die Reaktion wird vorzugsweise in Apparaten durchgeführt, die auch für die Sprühtrocknung geeignet sind. Derartige Reaktoren werden beispielsweise in K. Masters, Spray Drying Handbook, 5th Edition, Longman, 1991, Seiten 23 bis 66, beschrieben.

Im erfindungsgemäßen Verfahren können eine oder mehrere Sprühdüsen eingesetzt werden. Die einsetzbaren Sprühdüsen unterliegen keiner Beschränkung. Derartigen Düsen kann die zu versprühende Flüssigkeit unter Druck zugeführt werden. Die Zerteilung der zu versprühenden Flüssigkeit kann dabei dadurch erfolgen, dass sie nach Erreichen einer bestimmten Mindestgeschwindigkeit in der Düsenbohrung entspannt wird. Ferner können für den erfindungsgemäßen Zweck auch Einstoffdüsen, wie beispielsweise Schlitzdüsen oder Drallkammern (Vollkegeldüsen) verwendet werden (beispielsweise von Düsen-Schlick GmbH, DE, oder von Spraying Systems Deutschland GmbH, DE).

Erfindungsgemäß bevorzugt sind Vollkegeldüsen. Darunter sind solche mit einem Öffnungswinkel des Sprühkegels von 60 bis 180° bevorzugt. Besonders bevorzugt sind Öffnungswinkel von 90 bis 120°. Der sich beim Versprühen einstellende mittlere Tropfendurchmesser ist erfindungsgemäß typischerweise kleiner 1000 µm, vorzugsweise kleiner 200 µm, bevorzugt kleiner 100 µm, sowie üblicherweise größer 10 µm, vorzugsweise größer 20 µm, bevorzugt größer 50 µm, und kann nach üblichen Methoden, wie Lichtstreuung, oder anhand der bei den Düsenherstellern erhältlichen Kennlinien bestimmt werden. Der Durchsatz je Sprühdüse beträgt zweckmäßig 0,1 bis 10 m³/h, häufig 0,5 bis 5 m³/h.

Der sich beim Versprühen einstellende Tröpfchendurchmesser ist zweckmäßig von 10 bis 1.000 µm, bevorzugt von 10 bis 500 µm, besonders bevorzugt von 10 bis 150 µm, ganz besonders bevorzugt von 10 bis 45 µm.

Die Reaktion kann auch in Apparaten durchgeführt werden in denen die Monomerlösung in Form monodisperser Tropfen frei fallen kann. Geeignet dazu sind Apparaturen, wie sie beispielsweise in der Patentschrift US-A-5,269,980, Spalte 3, Zeilen 25 bis 32, beschrieben sind.

Eine Vertropfung durch laminaren Strahlzerfall, wie in Rev. Sci. Instr., Band 38 (1966), Seiten 502 bis 506, beschrieben, ist ebenfalls möglich.

Die Vertropfung ist gegenüber der Versprühung bevorzugt, insbesondere bei Verwendung von Photoinitiatoren. Durch Vertropfung werden im erfindungsgemäßen Verfahren polymere Verdicker mit niedrigem Staubanteil, optimaler Schüttdichte und guter Fließfähigkeit erhalten.

Sind dagegen hohe Durchsätze an Monomerlösung gewünscht, so ist das Versprühen der Monomerlösung in den Reaktionsraum bevorzugt.

Die Reaktionsraum des Polymeriationsreaktors kann im Überdruck oder im Unterdruck durchgeführt werden, ein Unterduck von bis zu 100 mbar gegenüber dem Umgebungsdruck ist bevorzugt.

Die Polymerisationsgeschwindigkeit und die Trockengeschwindigkeit weisen üblicherweise unterschiedliche Temperturabhängigkeiten auf. Dies kann beispielsweise bedeuten, dass die versprühten Tropfen trocknen bevor der gewünschte Umsatz erreicht worden ist. Daher ist es vorteilhaft die Reaktionsgeschwindigkeit und die Trockengeschwindigkeit getrennt zu beeinflussen.

Die Trockengeschwindigkeit kann über den Wasserdampfgehalt des Inertgases beeinflusst werden. Der Wasserdampfgehalt des Inertgases beträgt im allgemeinen bis 90 Vol.-%, vorzugsweise bis 50 Vol.-%.

Die Polymeriationsgeschwindigkeit kann durch Art und Menge des verwendeten Initiatorsystems eingestellt werden.

Vorteilhaft zur Steuerung der Polymerisationsgeschwindigkeit ist die Verwendung von Azoverbindungen oder Redoxinitiatoren als Initiatoren. Das Anspringverhalten der Polymerisation läßt sich mit Azoverbindungen oder Redoxinitiatoren über Auswahl des Initiators, Initiatorkonzentration und Reaktionstemperatur besser steuern als beispielsweise mit reinen Peroxidinitiatoren.

Besonders vorteilhaft sind Photoinitiatoren. Bei Verwendung von Photoinitiatoren kann die Trockengeschwindigkeit über die Temperatur auf den gewünschten Wert eingestellt werden, ohne dass damit gleichzeitig die Radikalbildung wesentlich beeinflusst wird.

Das Trägergas wird zweckmäßigerweise vor dem Reaktor auf die Reaktionstemperatur von 70 bis 250°C, vorzugsweise 80 bis 190°C, besonders bevorzugt 90 bis 160°C, vorgewärmt.

Das Reaktionsabgas, d.h. das der Reaktionsraum verlassende Trägergas, kann beispielsweise in einem Wärmeaustauscher abgekühlt werden. Dabei kondensieren Wasser und nicht umgesetztes Monomer. Danach kann das Reaktionsabgas zumindest teilweise wieder aufgewärmt und als Kreisgas in den Reaktor zurückgeführt werden. Vorzugsweise wird das Kreisgas so abgekühlt, dass das abgekühlte Kreisgas den für die Reaktion gewünschten Anteil an Wasserdampf hat. Ein Teil des Reaktionsabgases kann ausgeschleust und durch frisches Trägergas ersetzt werden, wobei im Reaktionsabgas enthaltene nicht umgesetzte Monomere abgetrennt und rückgeführt werden können.

Besonders bevorzugt ist ein Wärmeverbund, dass heißt, ein Teil der Abwärme beim Abkühlen des Abgases wird zum Aufwärmen des Kreisgases verwendet.

Die Reaktoren können begleitbeheizt werden. Die Begleitheizung wird dabei so eingestellt, dass die Wandtemperatur mindestens 5°C oberhalb der Reaktorinnentemperatur liegt und die Kondensation an den Reaktorwänden zuverlässig vermieden wird.

Das Reaktionsprodukt kann dem Reaktor in üblicher Weise entnommen werden, vorzugsweise am Boden über eine Förderschnecke, und gegebenenfalls bis zur gewünschten Restfeuchte und zum gewünschten Restmonomerengehalt getrocknet werden, beispielsweise in einer integrierten Wirbelschicht.

Ein weiterer Gegenstand der vorliegenden Erfindung sind die gemäß dem erfindungsgemäßen Verfahren erhältlichen wasserabsorbierenden Polymerpartikel.

Ein weiterer Gegenstand der vorliegenden Erfindung sind wasserabsorbierende Polymerpartikel, enthaltend
i) mindestens ein einpolymerisiertes ethylenisch ungesättigtes, säuregruppentragendes Monomer, dass gegebenenfalls zumindest teilweise neutralisiert sein kann,
ii) mindestens einen einpolymerisierten Vernetzer,
ii') mindestens einen weiteren, ein polymerisierten Vernetzer,
iii) gegebenenfalls ein oder mehrere einpolymerisierte mit den unter i) genannten Monomeren copolymerisierbare ethylenisch und/oder allylisch ungesättigte Monomere und
iv) gegebenenfalls ein oder mehrere wasserlösliche Polymere, auf die die unter i) genannten Monomere zumindest teilweise aufgeproft sind,
wobei die Konzentration des einpolymerisierten Vernetzers ii) im Bereich der Partikeloberfläche gegenüber dem Partikelinneren erhöht ist, und wobei für die Komponenten i) bis iv) sinngemäß das für die Komponenten a) bis d) obengenannte gilt, der mindestens eine einpolymerisierte Vernetzer ii) ein Vernetzer der allgemeinen Formel I 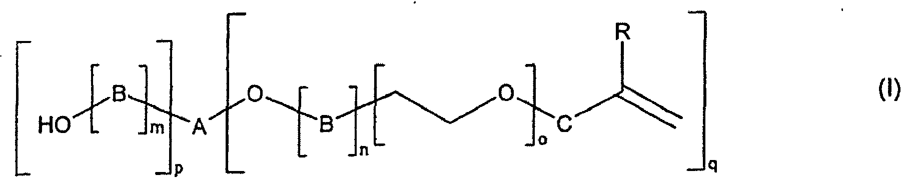 worin
- R: für gleiche und verschiedene Reste, ausgewählt aus der Gruppe Wasser-stoff und Methyl steht,
- A: ein C3- bis C10-Alk(p+q)yl oder C3- bis C10-Heteroalk(p+q)yl bedeutetet,
- B: für gleiche oder verschiedene Gruppen, ausgewählt aus 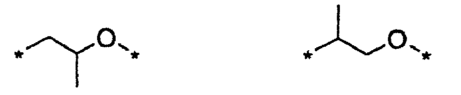
steht und mit * die Verknüpfungspositionen gekennzeichnet sind,
- C: für gleiche oder verschiedene Gruppen, ausgewählt aus Carbonyl und Methylen, steht,
- m, n und o: eine ganze Zahl von 1 bis 50 bedeuten,
- p: eine ganze Zahl von 0 bis 4 bedeutet und
- q: eine ganze Zahl von 2 bis 4 bedeutet,
oder ein Vernetzer der allgemeinen Formel II 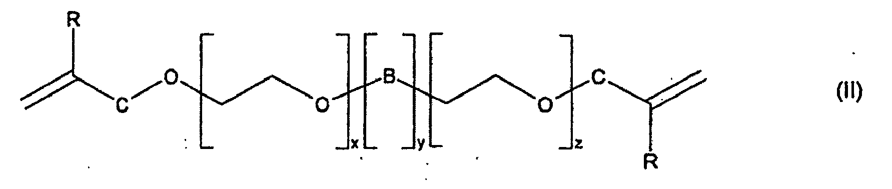 in der R, B und C die obengenannten Bedeutungen haben,
- x und z: gleich oder verschieden sind und eine ganze Zahl von 0 bis 100 be-deuten und
- y: eine ganze Zahl von 1 bis 200 bedeutet,
ist und der mindestens eine weitere einpolymerisierte Vernetzer ii') mindestens zwei polymerisierbare Gruppen aufwies, die in das Polymernetzwerk radikalisch einpolymerisiert werden können.

Die erhöhte Vernetzungsdichte im Bereich der Partikeloberfläche wird ohne einen zusätzlichen Nachvernetzungsschritt erreicht. Selbstverständlich können die nach dem erfindungsgemäßen Verfahren erhältlichen wasserabsorbierenden Polymerpartikel bei Bedarf noch zusätzlich nachvernetzt werden.

Kennzeichen für die erhöhte Vernetzungsdichte im Bereich der Partikeloberfläche sind eine hohe Zentrifugenretentionskapazität (CRC) und eine hohe Absorption unter Druck (AUL) der wasserabsorbierenden Polymerpartikel.

Die erfindungsgemäßen wasserabsorbierenden Polymerpartikel weisen typischerweise eine Zentrifugenretentionskapazität (CRC) von mindestens 15 g/g, vorzugsweise mindestens 20 g/g, besonders bevorzugt mindestens 25 g/g, auf. Die Zentrifugenretentionskapazität (CRC) wird gemäß der von der EDANA (European Disposables and Nonwovens Association) empfohlenen Testmethode Nr. 441.2-02 "Centrifuge retention capacity" bestimmt.

Die erfindungsgemäßen wasserabsorbierenden Polymerpartikel weisen typischerweise eine Absorption unter Druck 0,3 psi (2,07 kPa) von mindestens 10 g/g, vorzugsweise mindestens 15 g/g, besonders bevorzugt mindestens 20 g/g, auf. Die Absorption unter Druck (AUL) wird gemäß der von der EDANA (European Disposables and Nonwovens Association) empfohlenen Testmethode Nr. 442.2-02 "Absorption under pressure" bestimmt.

Ein weiterer Gegenstand der vorliegenden Erfindung sind Verfahren zur Herstellung von Hygieneartikeln, insbesondere Windeln, umfassend die Verwendung gemäß dem erfindungsgemäßen Verfahren hergestellter wasserabsorbierender Polymerpartikel.

Ein weiterer Gegenstand der vorliegenden Erfindung sind Hygieneartikel, enthaltend eine absorbierende Schicht bestehend aus 50 bis 100 Gew.-%, vorzugsweise 60 bis 100 Gew.-%, bevorzugt 70 bis 100 Gew.-%, besonders bevorzugt 80 bis 100 Gew.-%, ganz besonders bevorzugt 90 bis 100 Gew.-%, erfindungsgemäßer wasserabsorbierender Polymerpartikel, wobei die Umhüllung der absorbierenden Schicht selbstverständlich nicht berücksichtigt ist.

Zur Bestimmung der Güte der Nachvernetzung werden die getrocknete wasserabsorbierenden Polymerpartikel mit den nachfolgend beschrieben Testmethoden geprüft.

### Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymere werden vor der Messung gut durchmischt.

### Zentrifugenretentionskapazität (CRC Centrifuge Retention Capacity)

Die Zentrifugenretentionskapazität der wasserabsorbierenden Polymerpartikel wird gemäß der von der EDAN (European Disposables and Nonwovens Association) empfohlenen Testmethode Nr. 441.2-02 "Centrifuge retention capacity" bestimmt.

### Absorption unter Druck (AUL Absorbency Under Load)

Die Absorption unter Druck der wasserabsorbierenden Polymerpartikel wird gemäß der von der EDANA (European Disposables and Nonwovens Association) empfohlenen Testmethode Nr. 442.2-02 " Absorption under pressure" bestimmt.

### Extrahierbare

Der Gehalt an extrahierbaren Bestandteilen der wasserabsorbierenden Polymerpartikel wird gemäß der von der EDANA (European Disposables and Nonwovens Association) empfohlenen Testmethode Nr. 470.2-02 "Determination of extractable polymer content by potentiometric titration" bestimmt.

### Restmonomere

Der Gehalt an Restmonomeren der wasserabsorbierenden Polymerpartikel wird gemäß der von der EDANA (European Disposables and Nonwovens Association) empfohlenen Testmethode Nr. 410.2-02 "Residual monomers" bestimmt.

### mittlere Partikelgröße

Die mittlere Partikelgröße der wasserabsorbierenden Polymerpartikel wird gemäß der von der EDANA (European Disposables and Nonvowens Association) empfohlene Testmethode Nr. 420.2-02 "Particle size distribution" bestimmt.

Die EDANA-Testmethoden sind beispielsweise erhältlich bei der European Disposables and Nonwovens Association, Avenue Eugène Plasky 157, B-1030 Brüssel, Belgien.

### Beispiele:

### Beispiel 1 (Referenz)

300 g eines PO/EO-Blockpolymers, 12,9 g Acrylsäure, 0,3 g Hydrochinonmonomethylether, 0,09 g Kupfer-(II)-chlorid, 0,3 g hypophosphorige Säure, 0,1 g Triphenylphosphit, 300 g Cyclohexan und 4,5 g Schwefelsäure wurden in einem Rundkolben mit Wasserauskreiser vorgelegt. Innerhalb von 8 Stunden wurden bei einer Reaktionstemperatur von ca. 82°C insgesamt 7 ml Wasser ausgekreist. Anschließend wurde Cyclohexan abdestilliert. Der Vernetzer A wurde nicht weiter gereinigt.

Vernetzer A ist das Diacrylat eines Blockpolymers aus Propylenoxid und Ethylenoxid (allgemeine Formel II mit R = Wasserstoff, B = Propylenglykol, C = Carbonyl, y = ca. 15 und x+z = ca. 2).

### Beispiel 2 (Referenz)

300 g eines PO/EO-Blockpolymers, 7,4 g Acrylsäure, 0,3 g Hydrochinonmonomethylether, 0,09 g Kupfer-(II)-chlorid, 0,3 g hypophosphorige Säure, 0,1 g Triphenylphosphit, 300 g Cyclohexan und 4,5 g Schwefelsäure wurden in einem Rundkolben mit Wasserauskreiser vorgelegt. Innerhalb von 8 Stunden wurden bei iner Reaktionstemperatur von ca. 82°C insgesamt 6 ml Wasser ausgekreist. Anschließend wurde Cyclohexan abdestilliert. Der Vernetzer B wurde nicht weiter gereinigt.

Vernetzer B ist das Diacrylat eines Blockpolymers aus Propylenoxid und Ethylenoxid (allgemeine Formel II mit R = Wasserstoff, B = Propylenglykol, C = Carbonyl, y = ca. 19 und x+z = ca. 11).

### Beispiel 3 (Referenz)

300 g eines PO/EO-Blockpolymers, 3,1 g Acrylsäure, 0,3 g Hydrochinonmonomethylether, 0,09 g Kupfer-(II)-chlorid, 0,3 g hypophosphorige Säure, 0,1 g Triphenylphosphit, 300 g Cyclohexan und 4,5 g Schwefelsäure wurden in einem Rundkolben mit Wasserauskreiser vorgelegt. Innerhalb von 6 Stunden wurden bei iner Reaktions-temperatur von ca. 82°C insgesamt 3 ml Wasser ausgekreist. Anschließend wurde Cyclohexan abdestilliert. Der Vernetzer C wurde nicht weiter gereinigt.

Vernetzer C ist das Diacrylat eines Blockpolymers aus Propylenoxid und Ethylenoxid (allgemeine Formel II mit R = Wasserstoff, B = Propylenglykol, C = Carbonyl, y = ca. 30 und x+z = ca. 27).

### Beispiele 4 bis 8

Eine Lösung, enthaltend 25,2 kg Acrylsäure, 16,6 kg 50 gew.-%ige Natronlauge, 220 bzw. 255 g Vernetzer bzw. Vernetzergemisch, 55 kg Wasser und 1,8 kg 2,2'-Azobis(N,N'-dimethylenisobutyramidin)dihydrochlorid (Azoinitiator V44 der Wako Deutschland, DE) wurde in einem erwärmten, mit Stickstoffatmosphäre gefüllten Sprühturm (150°C, 12m Höhe, 2m Breite, Gasgeschwindigkeit 0,1 m/s im Gleichstrom) vertropft bzw. verdüst. Am Boden des Sprühturms wurde ein trockenes, weißes Pulver erhalten.

Die Versuchsbedingungen und die Versuchsergebnisse sind in den folgenden Tabellen zusammengefast:

**Tabelle 1: Versuchsbedingungen**

| Beispiel | Vernetzer 1 | Vernetzer 2 | Dosierung |
|---|---|---|---|
| 4* | 0,22 Gew.-% PEGDA 400 | | vertropft durch 150 µm Bohrung |
| 5 | 0,15 Gew.-% PEGDA 400 | 0,10 Gew.-% Vernetzer A aus Beispiel 1 | vertropft durch 150 µm Bohrung |
| 6 | 0,15 Gew.-% PEGDA 400 | 0,10 Gew.-% Vernetzer B aus Beispiel 2 | vertropft durch 150 µm Bohrung |
| 7 | 0,15 Gew.-% PEGDA 400 | 0,10 Gew.-% Vernetzer C aus Beispiel 3 | vertropft durch 150 µm Bohrung |
| 8 | 0,15 Gew.-% PEGDA 400 | 0,10 Gew.-% Vernetzer A aus Beispiel 1 | verdüst durch 400 µm Bohrung |

PEGDA 400 ist das Diacrylat eines Polyethylenglykols mit einer mittleren Molmasse von ca. 400 g/mol.

Die Gewichtsprozente beziehen sich auf die Gesamtlösung.

**Tabelle 2: Versuchsergebnisse**

| Beispiel | Extrahierbare | Restmonomer | CRC | AUL0.3psi | mittlere Partikelgöße |
|---|---|---|---|---|---|
| 4* | 18,2 % | 1,5 Gew.% | 19,1 g/g | 9,2 g/g | 220 µm |
| 5 | 19,0 % | 1,2 Gew.% | 22,5 g/g | 18,1 g/g | 218 µm |
| 6 | 17,5 % | 1,4 Gew.% | 21,2 g/g | 17,6 g/g | 210 µm |
| 7 | 18,0 % | 1,5 Gew.% | 20,3 g/g | 16,8 g/g | 235 µm |
| 8 | 22,0 % | 1,7 Gew.% | 15,5 g/g | 14,6 g/g | 80 µm |

| | | | | | |
|---|---|---|---|---|---|
| * nicht erfindungsgemäß | | | | | |

## Patentansprüche

1. Verfahren zur Herstellung wasserabsorbierender Polymerpartikel durch Sprühpolymerisation einer Monomerlösung, enthaltend
a) mindestens ein ethylenisch ungesättigtes, säuregruppentragendes Monomer,
b) mindestens einen Vernetzer,
b') mindestens einen weiteren Vernetzer,
c) gegebenenfalls ein oder mehrere mit dem Monomer a) copolymerisierbare ethylenisch und/oder allylisch ungesättigte Monomere und
d) gegebenenfalls ein oder mehrere wasserlösliche Polymere, auf die die Monomere a), b) und ggf. c) zumindest teilweise aufgepfropft werden können,
wobei die Monomerlösung versprüht wird, **dadurch gekennzeichnet, dass** sich der mindestens eine Vernetzer b) an der Tropfenoberfläche anreichert und der mindestens eine weitere Vernetzer b') mindestens zwei polymerisierbare Gruppen aufweist, die in das Polymernetzwerk radikalisch einpolymerisiert werden können.

2. Verfahren gemäß Anspruch 1, **dadurch gekennzeichnet, dass** der Vernetzer b) mindestens zwei radikalisch polymerisierbare Gruppen aufweist.

3. Verfahren gemäß Anspruch 1 oder 2, **dadurch gekennzeichnet, dass** der Vernetzer b) bei der Messung der dynamischen Oberflächenspannung gemäß ASTM D3825 in weniger als 10 Sekunden den Gleichgewichtszustand erreicht.

4. Verfahren gemäß einem der Ansprüche 1 bis 3, **dadurch gekennzeichnet, dass** der Vernetzer b) eine Verbindung der Formel 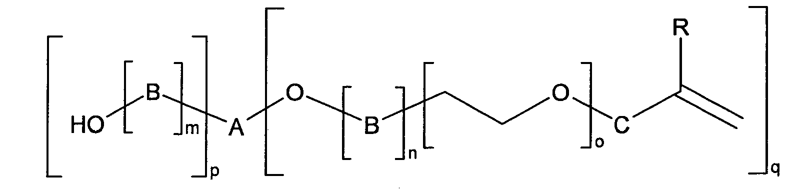 ist, worin
R für gleiche und verschiedene Reste, ausgewählt aus der Gruppe Wasser-stoff, Methyl und Ethyl steht,
A ein C₃- bis C₂₀-Alk(p+q)yl oder C₃- bis C₂₀-Heteroalk(p+q)yl bedeutetet,
B für gleiche oder verschiedene Gruppen, ausgewählt aus 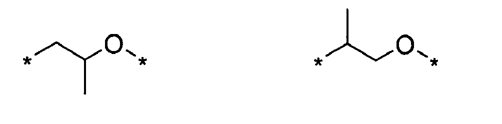 steht und mit * die Verknüpfungspositionen gekennzeichnet sind,
C für gleiche oder verschiedene Gruppen, ausgewählt aus Carbonyl und Methylen, steht,
m, n und o eine ganze Zahl von 0 bis 100 bedeuten,
p eine ganze Zahl von 0 bis 6 bedeutet und
q eine ganze Zahl von 2 bis 6 bedeutet.

5. Verfahren gemäß einem der Ansprüche 1 bis 4, **dadurch gekennzeichnet, dass** das Monomer a) Acrylsäure oder Methacrylsäure ist.

6. Verfahren gemäß einem der Ansprüche 1 bis 5, **dadurch gekennzeichnet, dass** das Monomer a) teilneutralisierte Acrylsäure oder Methacrylsäure ist.

7. Wasserabsorbierende Polymerpartikel, enthaltend
i) mindestens ein einpolymerisiertes ethylenisch ungesättigtes, säuregruppentragendes Monomer, das gegebenenfalls zumindest teilweise neutralisiert sein kann,
ii) mindestens einen einpolymerisierten Vernetzer,
ii') mindestens einen weiteren einpolymerisierten Vernetzer,
iii) gegebenenfalls ein oder mehrere einpolymerisierte mit den unter i) genannten Monomeren copolymerisierbare ethylenisch und/oder allylisch ungesättigte Monomere und
iv) gegebenenfalls ein oder mehrere wasserlösliche Polymere, auf die die unter i) genannten Monomere zumindest teilweise aufgepfropft sind,
wobei die Konzentration des einpolymerisierten Vernetzers ii) im Bereich der Partikeloberfläche gegenüber dem Partikelinneren erhöht ist, der mindestens eine einpolymerisierte Vernetzer ii) ein Vernetzer der allgemeinen Formel I 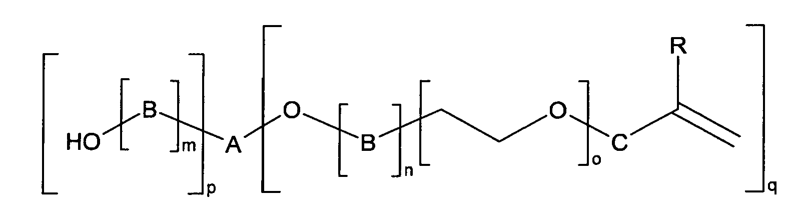 worin
R für gleiche und verschiedene Reste, ausgewählt aus der Gruppe Wasserstoff und Methyl steht,
A ein C₃- bis C₁₀-Alk(p+q)yl oder C₃- bis C₁₀-Heteroalk(p+q)yl bedeutetet,
B für gleiche oder verschiedene Gruppen, ausgewählt aus 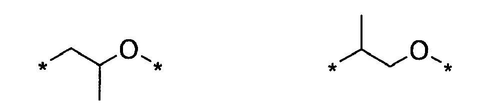 steht und mit * die Verknüpfungspositionen gekennzeichnet sind,
C für gleiche oder verschiedene Gruppen, ausgewählt aus Carbonyl und Methylen, steht,
m, n und o eine ganze Zahl von 1 bis 50 bedeuten,
p eine ganze Zahl von 0 bis 4 bedeutet und
q eine ganze Zahl von 2 bis 4 bedeutet,
oder ein Vernetzer der allgemeinen Formel II 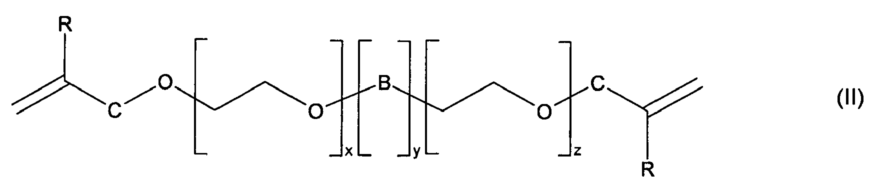 in der R, B und C die obengenannten Bedeutungen haben,
x und z gleich oder verschieden sind und eine ganze Zahl von 0 bis 100 bedeuten und
y eine ganze Zahl von 1 bis 200 bedeutet,
ist und der mindestens eine weitere einpolymerisierte Vernetzer ii') mindestens zwei polymerisierbare Gruppen aufwies, die in das Polymernetzwerk radikalisch einpolymerisiert werden können.

8. Polymerpartikel gemäß Anspruch 7, wobei der einpolymerisierte Vernetzer ii) radikalisch einpolymerisiert wurde.

9. Hygieneartikel, enthaltend wasserabsorbierende Polymerpartikel gemäß Anspruch 7 oder 8.

10. Hygieneartikel gemäß Anspruch 9, wobei der Hygieneartikel eine Windel ist.

## Claims

1. A process for producing water-absorbing polymeric particles by spray polymerization of a monomer solution comprising
a) at least one ethylenically unsaturated acid-functional monomer,
b) at least one crosslinker,
b') at least one further crosslinker,
c) optionally one or more ethylenically and/or allylically unsaturated monomers copolymerizable with the monomer a), and
d) optionally one or more water-soluble polymers whereonto the monomers a), b) and, if used, c) are at least partly graftable,
being spray dispensed, wherein the at least one crosslinker b) accumulates at the droplet surface and the at least one further crosslinker b') has at least two polymerizable groups which are free-radically polymerizable into the polymer network.

2. The process according to claim 1 wherein the crosslinker b) comprises at least two free-radically polymerizable groups.

3. The process according to claim 1 or 2 wherein the crosslinker b) equilibrates in less than 10 seconds in an ASTM D3825 measurement of dynamic surface tension.

4. The process according to any one of claims 1 to 3 wherein the crosslinker b) is a compound of the formula 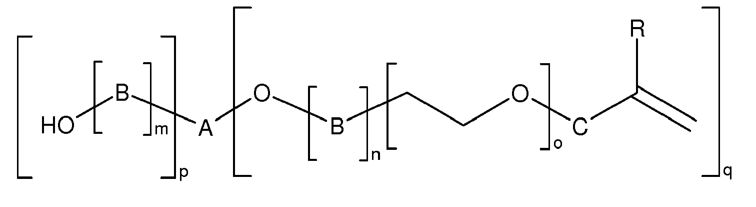 where
R represents the same or different radicals selected from the group consisting of hydrogen, methyl and ethyl,
A represents a C₃- to C₂₀-alk(p+q)yl or C₃- to C₂₀-heteroalk(p+q)yl,
B represents the same or different groups selected from 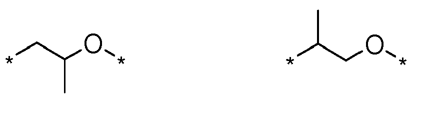 and * designates the positions of attachment,
C represents the same or different groups selected from carbonyl and methylene,
m, n and o are each an integer from 0 to 100,
p is an integer from 0 to 6, and
q is an integer from 2 to 6.

5. The process according to any one of claims 1 to 4 wherein the monomer a) is acrylic acid or methacrylic acid.

6. The process according to any one of claims 1 to 5 wherein the monomer a) is partially neutralized acrylic acid or methacrylic acid.

7. Water-absorbing polymeric particles comprising
i) at least one polymerized ethylenically unsaturated acid-functional monomer which optionally may be at least partially neutralized,
ii) at least one polymerized crosslinker,
ii') at least one further polymerized crosslinker,
iii) optionally one or more polymerized ethylenically and/or allylically unsaturated monomers copolymerizable with the monomers identified under i), and
iv) optionally one or more water-soluble polymers whereonto the monomers identified under i) are at least partly grafted,
wherein the concentration of the polymerized crosslinker ii) is higher in the region of the particle surface than in the particle interior, the at least one polymerized crosslinker ii) is a crosslinker of general formula I 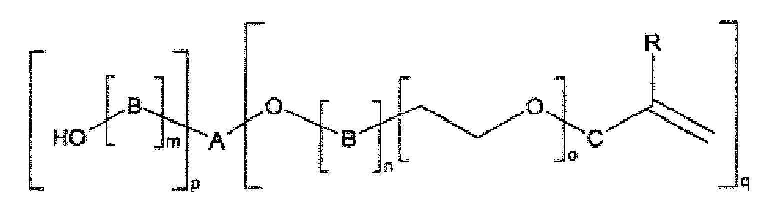 where
R represents the same or different radicals selected from the group consisting of hydrogen and methyl,
A represents a C₃- to C₁₀-alk(p+q)yl or C₃- to C₁₀-heteroalk(p+q)yl,
B represents the same or different groups selected from 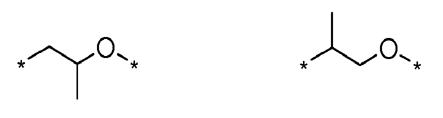 and * designates the positions of attachment,
C represents the same or different groups selected from carbonyl and methylene,
m, n and o are each an integer from 1 to 50,
p is an integer from 0 to 4, and
q is an integer from 2 to 4,
or a crosslinker of general formula II 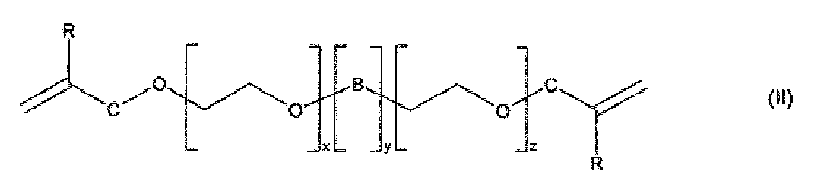 where R, B and C are each as defined above,
x and z are the same or different and are each an integer from 0 to 100, and
y is an integer from 1 to 200,
and the at least one further polymerized crosslinker ii') had at least two polymerizable groups which are free-radically polymerizable into the polymer network.

8. The polymeric particles according to claim 7 wherein the polymerized crosslinker ii) was free-radically polymerized.

9. A hygiene article comprising water-absorbing polymeric particles according to claim 7 or 8.

10. The hygiene article according to claim 9 which is a diaper.

## Revendications

1. Procédé pour la production de particules de polymère absorbant l'eau, par polymérisation par pulvérisation d'une solution de monomères, contenant
a) au moins un monomère à insaturation éthylénique, portant des groupes acides,
b) au moins un agent de réticulation,
b') au moins un autre agent de réticulation,
c) éventuellement un ou plusieurs monomères à insaturation éthylénique et/ou allylique, copolymérisables avec le monomère a) et
d) éventuellement un ou plusieurs polymères hydrosolubles, sur lesquels peuvent être au moins partiellement greffés les monomères a), b) et éventuellement c),
la solution de monomères étant pulvérisée, **caractérisé en ce que** ledit au moins un agent de réticulation b) s'accumule à la surface des gouttes et l'au moins un autre agent de réticulation b') présente au moins deux groupes polymérisables qui peuvent être incorporés par polymérisation par voie radicalaire dans le réseau polymère.

2. Procédé selon la revendication 1, **caractérisé en ce que** l'agent de réticulation b) comprend au moins deux groupes polymérisables par voie radicalaire.

3. Procédé selon la revendication 1 ou 2, **caractérisé en ce que** dans la mesure de la tension superficielle dynamique selon ASTM D3825 l'agent de réticulation b) atteint l'état d'équilibre en moins de 10 secondes.

4. Procédé selon l'une quelconque des revendications 1 à 3, **caractérisé en ce que** l'agent de réticulation b) est un composé de formule 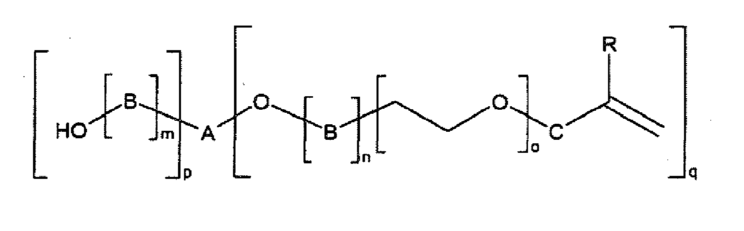 dans laquelle
R représente des radicaux identiques ou différents, choisis dans le groupe constitué par l'atome d'hydrogène, les groupes méthyle et éthyle,
A représente un groupe alk(p+q)yle en C₃-C₂₀ ou hétéroalk(p+q)yle en C₃-C₂₀,
B représente des groupes identiques ou différents, choisis parmi 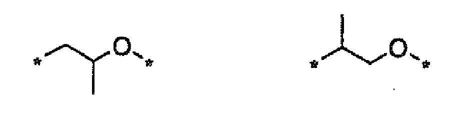 et les positions de liaison sont indiquées par *,
C représente des groupes identiques ou différents, choisis parmi les groupes carbonyle et méthylène,
m, n et o représentent un nombre entier valant de 0 à 100,
p représente un nombre entier valant de 0 à 6 et
q représente un nombre entier valant de 2 à 6.

5. Procédé selon l'une quelconque des revendications 1 à 4, **caractérisé en ce que** le monomère a) est l'acide acrylique ou l'acide méthacrylique.

6. Procédé selon l'une quelconque des revendications 1 à 5, **caractérisé en ce que** le monomère a) est un acide acrylique acide méthacrylique partiellement neutralisé.

7. Particules de polymère absorbant l'eau, contenant
i) au moins un monomère à insaturation éthylénique, portant des groupes acides, incorporé par polymérisation, qui peut éventuellement être au moins partiellement neutralisé,
ii) au moins un agent de réticulation incorporé par polymérisation,
ii') au moins un autre agent de réticulation incorporé par polymérisation,
iii) éventuellement un ou plusieurs monomères à insaturation éthylénique et/ou allylique, copolymérisables avec les monomères nommés en i), incorporés par polymérisation et
iv) éventuellement un ou plusieurs polymères hydrosolubles, sur lesquels peuvent être au moins partiellement greffés les monomères nommés en i),
la concentration de l'agent de réticulation ii) incorporé par polymérisation, dans la zone de la surface des particules, étant supérieure à celle à l'intérieur des particules, l'au moins un agent de réticulation incorporé par polymérisation ii) étant un agent de réticulation de formule générale I 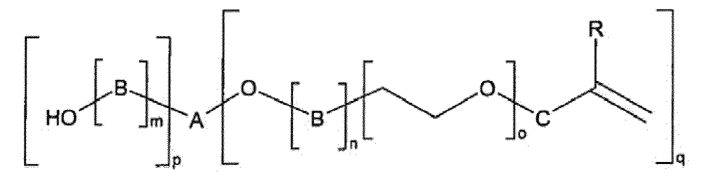 dans laquelle
R représente des radicaux identiques et différents, choisis dans le groupe constitué par l'atome d'hydrogène et le groupe méthyle,
A représente un groupe alk(p+q)yle en C₃-C₁₀ ou hétéroalk (p+q) yle en C₃-C₁₀,
B représente des groupes identiques ou différents, choisis parmi 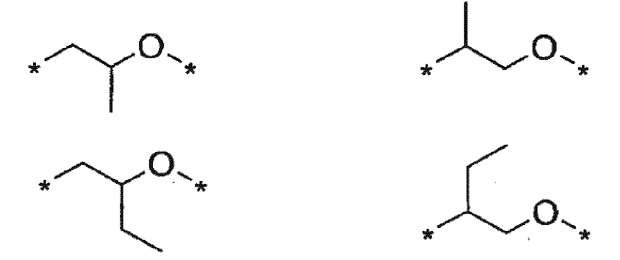
C et les positions de liaison sont indiquées par *, représente des groupes identiques ou différents, choisis parmi les groupes carbonyle et méthylène,
m, n et o représentent un nombre entier valant de 1 à 50,
p représente un nombre entier valant de 0 à 4, et
q représente un nombre entier valant de 2 à 4,
ou un agent de réticulation de formule générale II 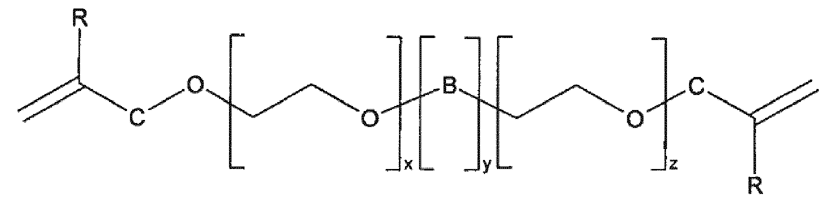 dans laquelle R, B et C ont les significations susmentionnées,
x et z sont identiques ou différents, et représentent un nombre entier valant de 0 à 100 et
y représente un nombre entier valant de 1 à 200,
et l'au moins un autre agent de réticulation incorporé par polymérisation ii') présente au moins deux groupes polymérisables qui peuvent être incorporés par polymérisation par voie radicalaire dans le réseau polymère.

8. Particules de polymère selon la revendication 7, l'agent de réticulation ii) incorporé par polymérisation ayant été incorporé par polymérisation radicalaire.

9. Article d'hygiène, contenant des particules de polymère absorbant l'eau selon la revendication 7 ou 8.

10. Article d'hygiène selon la revendication 9, l'article d'hygiène étant une couche.
